# EUROPEAN PATENT APPLICATION

(11) **EP 1 985 627 A1**
(43) Date of publication of application: **29.10.2008**
(21) Application number: 07008661.6
(22) Date of filing: 27.04.2007
(51) Int. Cl.: C07K 14/435

(54) **Putative chloroplast transporter as novel specific kanamycin resistance marker**

(71) Applicant: Gregor Mendel Institute of Molecular Plant Biology, 1030 Wien (AT); Austria Wirtschaftsservice Gesellschaft m.b.H., 1030 Wien (AT)
(72) Inventor: Aufsatz, Werner, 5023 Salzburg-Gnigl (AT); Matzke, Marjori, 1140 Wien (AT); Matzke, Antonius, 1140 Wien (AT)
(74) Representative: Krauss, Jan

(57) **Abstract**

The present invention relates to a new kanamycin *Arabidopsis*-derived resistance marker, the At5g26820 gene, and respective uses thereof.

SEQUENCE LISTING

<110>  Gregor Mendel Institute of Molecular Plant Biology
       Austria Wirtschaftsservice Gesellschaft mbH

<120>  Putative chloroplast transporter as novel specific kanamycin
       resistance marker

<130>  FB18292

<160>  4

<170>  PatentIn version 3.4

<210>  1
<211>  2131
<212>  DNA
<213>  Arabidopsis thaliana

<400>  1
ggcgtgcttc gcgacgaacc gccacagata aggttcttct ttttttttcc aatatctctg      60

gatttttttt cccctcgttg aagaaaatct agtggtgatt aagccatagg ggaaacaaaa     120

cccagagaga cgagtttgct agattttttt tttttttgga ggctgcatga gtttggagaa     180

gaaggcgagt agtacaaatt acaactccag aaaggagaag aggagaatca atttatagaa     240

tggttgtttc aatggctttg gtcagacatt ctccgtcttt cgattttctg tttcattttc     300

cggtcgacag aagccgtttt ctatcgccgg ttgctttttc tagcgttcgc tatcatcgct     360

tccattcttg tagatggtta agtctccgct cttcaccttc atgttctcgc aggttaaaca     420

gttttagttc gaggtgttcg attacaaaca ctgatgtatg tcacgaattt gtgactactg     480

atgatgaaat ccacgaggat ttgcttactc caattgagga tcattcaatt cctattgttc     540

atcttgatac caatatctcc gttactgaat ctctgacctt actcaccgag tgtacttacg     600

tagatactgt tttaacagca ttgcctgtat tgtctgagga agagcagact gtaattgcag     660

ccactcctgc tcaccctgaa ggattatatg tcttatatgc aagttgtttg gttggaaatt     720

tggttgagca actttggaat tttgcttggc cgtccgccat tgcaatgcta tatccgagct     780

tactacctgt ggcagtcatg gggtttgtca ctaaattggc gataattgct ggaggccctg     840

tggttggaaa gtttatggat tatagtccga gagttcctac atatatctcg ttgaatgtca     900

ttcaggcagc cgctcaagtg ctatctgcag gaatgataat tcatgcatac acggttcctt     960

ccacatcggc gtcatcaatt cttctgcagc cttggttttt tgcattgtta ttcgcagggg    1020

ccattgacag tctatgtgga atagcatctg gagtcgccat tgaacgtgat tgggttgtat    1080

tgttggccgg cataaacaga ccaatcgctc ttgctcaagc aaatgctgtt ctccacagaa    1140

tagatctgct ttgcgagata gctgggacga tgttatttgg cattctccta tccaaatatg    1200

accctgtgac ctgcttaaag tttgctgcca cactaatggt gggttctttg ccaaccatga    1260

cagctcttat atggctgacc aacaagttct cctctggagt tctggaccgt cctaaatgct    1320

ccttgaacag ctgttctgct gaaggatctc gtactaatac cgacagtatt tttgatattg 1380

gcatggaaac tatcaagctc ggatggaaag aatatattca acagccagtt cttcctgcta 1440

gcctcgcata tgtccttctc tactttaaca tcgtcctcac cccaggcagc ttgatgactg 1500

catttctaac acaacgatgt gtgaatccat cagttattgg tggtttcagt ggattatgcg 1560

ctgttatggg cgtcgccgca accttcttat cagcaaactt ggtcaaacga gttggcattt 1620

taaaggcggg tgcagtagga ttgtttttcc aagcttcact ccttgccgtt gctgttgctg 1680

tgtattgtag ctcttctcta tcccataaaa gcccactctt cttcttcttg tccatgatcg 1740

tattatcaag gctaggtcac atgtcttacg gcgttgtggg agctcaaata cttcaaaccg 1800

gcattccatc atccaaggct aatctcatcg gcgcaacgga gatctcagtg gcaagtctag 1860

ctgaatcgct tatgctcgga gtagccatag cggcgaatga cgcttcccat ttcgggtttc 1920

tcgcggttct gtctctttta tcagttgttg cggcctcatt gatattctgc agattgctga 1980

gaaatcccac tgatgaacaa agacgactct tctccttcga ccctctctca aattgatttg 2040

gagcttttac attgaaatat ttgtatagta gtttcacttt cagtgtataa attctttata 2100

taaagtaaag aaaaaaattg actttggtgt g 2131

<210> 2
<211> 598
<212> PRT
<213> Arabidopsis thaliana

<400> 2

Met Val Val Ser Met Ala Leu Val Arg His Ser Pro Ser Phe Asp Phe
1               5                   10                  15

Leu Phe His Phe Pro Val Asp Arg Ser Arg Phe Leu Ser Pro Val Ala
            20                  25                  30

Phe Ser Ser Val Arg Tyr His Arg Phe His Ser Cys Arg Trp Leu Ser
        35                  40                  45

Leu Arg Ser Ser Pro Ser Cys Ser Arg Arg Leu Asn Ser Phe Ser Ser
    50                  55                  60

Arg Cys Ser Ile Thr Asn Thr Asp Val Cys His Glu Phe Val Thr Thr
65                  70                  75                  80

Asp Asp Glu Ile His Glu Asp Leu Leu Thr Pro Ile Glu Asp His Ser
                85                  90                  95

Ile Pro Ile Val His Leu Asp Thr Asn Ile Ser Val Thr Glu Ser Leu
            100                 105                 110

Thr Leu Leu Thr Glu Cys Thr Tyr Val Asp Thr Val Leu Thr Ala Leu
        115                 120                 125

Pro Val Leu Ser Glu Glu Glu Gln Thr Val Ile Ala Ala Thr Pro Ala
    130                 135                 140

His Pro Glu Gly Leu Tyr Val Leu Tyr Ala Ser Cys Leu Val Gly Asn
145                 150                 155                 160

Leu Val Glu Gln Leu Trp Asn Phe Ala Trp Pro Ser Ala Ile Ala Met
                165                 170                 175

Leu Tyr Pro Ser Leu Leu Pro Val Ala Val Met Gly Phe Val Thr Lys
            180                 185                 190

Leu Ala Ile Ile Ala Gly Gly Pro Val Val Gly Lys Phe Met Asp Tyr
        195                 200                 205

Ser Pro Arg Val Pro Thr Tyr Ile Ser Leu Asn Val Ile Gln Ala Ala
    210                 215                 220

Ala Gln Val Leu Ser Ala Gly Met Ile Ile His Ala Tyr Thr Val Pro
225                 230                 235                 240

Ser Thr Ser Ala Ser Ser Ile Leu Leu Gln Pro Trp Phe Phe Ala Leu
                245                 250                 255

Leu Phe Ala Gly Ala Ile Asp Ser Leu Cys Gly Ile Ala Ser Gly Val
            260                 265                 270

Ala Ile Glu Arg Asp Trp Val Val Leu Leu Ala Gly Ile Asn Arg Pro
        275                 280                 285

Ile Ala Leu Ala Gln Ala Asn Ala Val Leu His Arg Ile Asp Leu Leu
    290                 295                 300

Cys Glu Ile Ala Gly Thr Met Leu Phe Gly Ile Leu Leu Ser Lys Tyr
305                 310                 315                 320

Asp Pro Val Thr Cys Leu Lys Phe Ala Ala Thr Leu Met Val Gly Ser
                325                 330                 335

Leu Pro Thr Met Thr Ala Leu Ile Trp Leu Thr Asn Lys Phe Ser Ser
            340                 345                 350

Gly Val Leu Asp Arg Pro Lys Cys Ser Leu Asn Ser Cys Ser Ala Glu

355                 360                 365

Gly Ser Arg Thr Asn Thr Asp Ser Ile Phe Asp Ile Gly Met Glu Thr
    370                 375                 380

Ile Lys Leu Gly Trp Lys Glu Tyr Ile Gln Gln Pro Val Leu Pro Ala
385                 390                 395                 400

Ser Leu Ala Tyr Val Leu Leu Tyr Phe Asn Ile Val Leu Thr Pro Gly
                405                 410                 415

Ser Leu Met Thr Ala Phe Leu Thr Gln Arg Cys Val Asn Pro Ser Val
            420                 425                 430

Ile Gly Gly Phe Ser Gly Leu Cys Ala Val Met Gly Val Ala Ala Thr
        435                 440                 445

Phe Leu Ser Ala Asn Leu Val Lys Arg Val Gly Ile Leu Lys Ala Gly
    450                 455                 460

Ala Val Gly Leu Phe Phe Gln Ala Ser Leu Leu Ala Val Ala Val Ala
465                 470                 475                 480

Val Tyr Cys Ser Ser Ser Leu Ser His Lys Ser Pro Leu Phe Phe Phe
                485                 490                 495

Leu Ser Met Ile Val Leu Ser Arg Leu Gly His Met Ser Tyr Gly Val
            500                 505                 510

Val Gly Ala Gln Ile Leu Gln Thr Gly Ile Pro Ser Ser Lys Ala Asn
        515                 520                 525

Leu Ile Gly Ala Thr Glu Ile Ser Val Ala Ser Leu Ala Glu Ser Leu
    530                 535                 540

Met Leu Gly Val Ala Ile Ala Ala Asn Asp Ala Ser His Phe Gly Phe
545                 550                 555                 560

Leu Ala Val Leu Ser Leu Leu Ser Val Val Ala Ala Ser Leu Ile Phe
                565                 570                 575

Cys Arg Leu Leu Arg Asn Pro Thr Asp Glu Gln Arg Arg Leu Phe Ser
            580                 585                 590

Phe Asp Pro Leu Ser Asn
        595

<210> 3
<211> 2220
<212> DNA
<213> Oryza sativa

<400> 3
tcatccatat tcgtgctggg ctgtaaggag ggcccagccc aatatagaag tataatactc 60

ggcccaaata aatcttcttc cctatccatc cccaactccc tatccgttcc agctcatcag 120

ctcactcccg cctccatgtc catgtccaag ctcctctcgc cgcctcccac ctcaccacca 180

ggaccagccc tctcccggct cccgtgtcgc cgtgtagcgc cgcctcccgt gctgcccttc 240

ccctttcctc tccgccgctt gacgtcgcgc cgcgtcttcg ccaccagctg ctcctcctcc 300

gactccgaac atgctccttc ggcctccagc accgcccttg ccggcgccgg agacgacctc 360

tccgccggtg taacccaaga acgcgaaggc gcactgccgt ttgttcagct ctcctcgggt 420

atagtcctcc ggaccgagga gcagagcttg ctcggggacc acgcgccggc gccggcgccg 480

gcctccgccg cttcgtcttt cgcgttgctg gatgagctca acggaggctg cagagaggat 540

gaccacctgg gagaaactcc ggcttaccca gccgccatga acggtacgaa ttctgcttct 600

ctggagctca attgttgcat ctctcctgaa tcgtgttcga cgcgcagcgc tgtacgcggc 660

ctgcctcgcc gggaacgcga cggagcagct ctggaacttc acatggccgg ccgccgtcgc 720

cgtgctccac ccggcgagca tcctccccgt cgccgtcctc ggcttcttca ccaaggtgca 780

gaacgccgac cactgactct ccaactaata tatgcgttcg aattaatggc gacattctga 840

cgaaatccga tttaattttg cgtgctagct cgtcgtgttc gccgccggcc cgttggtcgg 900

cgagctcatc agctcgctcc cacgcatccc cgcctaccga tctctcgccg caattcaggt 960

gactatccat gatccgaaaa agcaacgtca gatttctgct tcaagaagag tgtcatcaac 1020

atcatcgtgt cgtgccagac cgcggcgcat ttggtgtcgg tggcgacgat cacctacgcg 1080

ttcgccgtcc acagagcggc ggcggcatcg ctgctcctgc ggccgtggtt cgccgtgctg 1140

gtggcgtcca ccgccgtcga caggctcgcc tgcgtcgcgc tcggcatcat cgcggagcgg 1200

gacttcgtcg tgcaggtgac cgaagctccc aaccacctcc tgccattgat tacatatata 1260

gctcgtcgtt gctttcttca acctctctcc gatgtttctc caatctccat ggatcgtgtg 1320

gttgaagcta gcaggggcag gcaggccggt ggcgctcgcc aaggccaacg cgacgctcag 1380

cagagtcgat ctcctctgcg aggtttggga tttcagaatt tctgcatctg aatcatcttt 1440

tcttgattat gaaatatgaa aaatgcatgt actatgcgtg tgttcgtgat gaatttcgtg 1500

tgatttgcag acggtaggag catccatctt cgcgttgctg ctctccaaga acaacccact 1560

cacctgcatc aagctatcct gcgtcatctc gctctgtgcc ctgcctctgc tggtacacta 1620

gctacagatt cataaattca gaaaattatt ttgcagattt agtacattat cattcaccag 1680

ttcaggaaaa gaaaatctat ggttctaatt tcgcagattt tcctgtgtgg agagatgaac 1740

cggctcgctg acggcatctt cgatcactct gaaaatacaa cctcacacgc tgaaaaaact 1800

tccagtttca gcatacgaaa aaccggtgat tcattgagct tgcagcctag ttctacgaca 1860

gttcatcgat cactatcaga tattcaaatt ggagaatttt ttttttgttt tttttttgcc 1920

aaaatctgat tttcgaattg tttgatcaat cctcatcttt gacagtggaa gaggctgtgg 1980

ctaccgtcag gaatggctgg agcgagtaca tgcggcagcc ggtgctcccg gcgagcctcg 2040

cctacgtgtt cgtctgcttc aacgtcgccc tcgcccccgg cgcgctcatg accaccttcc 2100

tcatccacca gggtgagatg acgcgcgcgc atggttacca ttgctgacat catcatcatt 2160

catcaagctc atcttgttaa tggggtgtgt gtgtgtggtg tgggcaggcg tgaggccgtc 2220

<210> 4
<211> 579
<212> PRT
<213> Oryza sativa

<400> 4

Met Ser Met Ser Lys Leu Leu Ser Pro Pro Pro Thr Ser Pro Pro Gly
1               5                   10                  15

Pro Ala Leu Ser Arg Leu Pro Cys Arg Arg Val Ala Pro Pro Pro Val
            20                  25                  30

Leu Pro Phe Pro Phe Pro Leu Arg Arg Leu Thr Ser Arg Arg Val Phe
        35                  40                  45

Ala Thr Ser Cys Ser Ser Ser Asp Ser Glu His Ala Pro Ser Ala Ser
    50                  55                  60

Ser Thr Ala Leu Ala Gly Ala Gly Asp Asp Leu Ser Ala Gly Val Thr
65                  70                  75                  80

Gln Glu Arg Glu Gly Ala Leu Pro Phe Val Gln Leu Ser Ser Gly Ile
                85                  90                  95

Val Leu Arg Thr Glu Glu Gln Ser Leu Leu Gly Asp His Ala Pro Ala
            100                 105                 110

Pro Ala Pro Ala Ser Ala Ala Ser Ser Phe Ala Leu Leu Asp Glu Leu
        115                 120                 125

Asn Gly Gly Cys Arg Glu Asp Asp His Leu Gly Glu Thr Pro Ala Tyr
    130                 135                 140

Pro Ala Ala Met Asn Ala Leu Tyr Ala Ala Cys Leu Ala Gly Asn Ala
145                 150                 155                 160

Thr Glu Gln Leu Trp Asn Phe Thr Trp Pro Ala Ala Val Ala Val Leu
                165                 170                 175

His Pro Ala Ser Ile Leu Pro Val Ala Val Leu Gly Phe Phe Thr Lys
            180                 185                 190

Leu Val Val Phe Ala Ala Gly Pro Leu Val Gly Glu Leu Ile Ser Ser
        195                 200                 205

Leu Pro Arg Ile Pro Ala Tyr Arg Ser Leu Ala Ala Ile Gln Lys Ser
    210                 215                 220

Val Ile Asn Ile Ile Val Ser Cys Gln Thr Ala Ala His Leu Val Ser
225                 230                 235                 240

Val Ala Thr Ile Thr Tyr Ala Phe Ala Val His Arg Ala Ala Ala Ala
                245                 250                 255

Ser Leu Leu Leu Arg Pro Trp Phe Ala Val Leu Val Ala Ser Thr Ala
            260                 265                 270

Val Asp Arg Leu Ala Cys Val Ala Leu Gly Ile Ile Ala Glu Arg Asp
        275                 280                 285

Phe Val Val Gln Thr Val Gly Ala Ser Ile Phe Ala Leu Leu Leu Ser
    290                 295                 300

Lys Asn Asn Pro Leu Thr Cys Ile Lys Leu Ser Cys Val Ile Ser Leu
305                 310                 315                 320

Cys Ala Leu Pro Leu Leu Ile Phe Leu Cys Gly Glu Met Asn Arg Leu
                325                 330                 335

Ala Asp Gly Ile Phe Asp His Ser Glu Asn Thr Thr Ser His Ala Glu
            340                 345                 350

Lys Thr Ser Ser Phe Ser Ile Arg Lys Thr Val Glu Glu Ala Val Ala
        355                 360                 365

Thr Val Arg Asn Gly Trp Ser Glu Tyr Met Arg Gln Pro Val Leu Pro
    370                 375                 380

Ala Ser Leu Ala Tyr Val Phe Val Cys Phe Asn Val Ala Leu Ala Pro
385                 390                 395                 400

Gly Ala Leu Met Thr Thr Phe Leu Ile His Gln Gly Val Arg Pro Ser

                405                 410                 415

Val Ile Gly Ala Phe Gly Gly Ser Ser Gly Ala Val Gly Ile Leu Ala
            420                 425                 430

Thr Phe Ala Thr Ala Arg Leu Val Lys Glu Leu Gly Ile Leu Lys Ala
        435                 440                 445

Gly Ala Ala Gly Leu Ile Ala Gln Ser Ala Leu Leu Gly Ala Ala Val
    450                 455                 460

Val Val Tyr Leu Thr Gly Ala Val Ser Arg Arg Ala Gly Ala Leu Phe
465                 470                 475                 480

Ala Phe Leu Gly Leu Ile Val Ala Ser Arg Ala Gly His Met Ala Tyr
                485                 490                 495

Ser Ala Ile Gly Leu Gln Val Val Gln Thr Gly Asn Pro Ala Ser Lys
            500                 505                 510

Ala Lys Leu Ile Gly Ala Thr Glu Ile Ala Val Ala Ser Leu Ala Glu
        515                 520                 525

Leu Ala Met Met Ala Val Ala Val Val Ala Ser Asp Ala Ser His Phe
    530                 535                 540

Gly Ala Leu Ala Ala Leu Ser Ala Thr Ala Val Thr Ala Ala Ala Gly
545                 550                 555                 560

Met Tyr Cys Arg Trp Leu Ala Asn Pro Ser Asp Glu Leu Arg Arg Ile
                565                 570                 575

Phe Pro Ser

## Description

The present invention relates to a new kanamycin *Arabidopsis-derived* resistance marker, the At5g26820 gene, and respective uses thereof.

### Description

Antibiotic resistance markers provide important and valuable tools for the generation of genetically modified organisms, such as plants and animals. Many of these markers are known and are used on a very widespread basis. Many of the markers provide for a "positive selection", i.e. the cells or organisms which carry and express such a marker (in many instances a foreign and thus "transgenically" provided gene) are able to survive a threat (selection) of an otherwise harmful substance, such as an antibiotic.

Antibiotic resistance markers are mainly used in the laboratory environment, since the use of antibiotic resistance genes outside the laboratory is very controversial in public and is used as a heavy argument against "green biotechnology", since the horizontal gene transfer of said markers from transgenic plants to bacteria and, ultimately, the generation of antibiotic resistant pathogenic bacteria could take place (as discussed, for example, in Nap JP, Bijvoet J, Stiekema WJ. Biosafety of kanamycin-resistant transgenic plants. Transgenic Res. 1992 Nov;1(6):239-49. and Goldstein DA, Tinland B, Gilbertson LA, Staub JM, Bannon GA, Goodman RE, McCoy RL, Silvanovich A. Human safety and genetically modified plants: a review of antibiotic resistance markers and future transformation selection technologies. J Appl Microbiol. 2005; 99(1):7-23).

As a result of the above, the farming of transgenic plants is heavily regulated by the respective national authorities. Furthermore, farmers that are using transgenic plants are facing legal problems with respect to liability in case of the spread of resistance markers.

Recently, Luo et al. (in Luo K, Duan H, Zhao D, Zheng X, Deng W, Chen Y, Stewart CN Jr, McAvoy R, Jiang X, Wu Y, He A, Pei Y, Li Y. 'GM-gene-deletor': fused loxP-FRT recognition sequences dramatically improve the efficiency of FLP or CRE recombinase on transgene excision from pollen and seed of tobacco plants. Plant Biotechnol J. 2007 Mar; 5(2):263-274.) reported a 'genetically modified (GM)-gene-deletor' system to remove all functional transgenes from pollen, seed or both. The 'GM-gene-deletor' reported may be used to produce 'non-transgenic' pollen and/or seed from transgenic plants and to provide a bioconfinement tool for transgenic crops and perennials, with special applicability towards vegetatively propagated plants and trees.

Kanamycin is a member of the aminoglycoside family of antibiotics, which specifically interfere with the function of prokaryotic ribosomes, but leave eukaryotic ribosomes relatively unaffected. Ribosomes in chloroplasts are of the prokaryotic type and thus are sensitive to these antibiotics. Thus, kanamycin resistance is one of the most frequently used selection markers for obtaining transgenic plants.

In view of the above, it is an object of the present invention, to provide novel genetic markers that provide kanamycin resistance in plants, and, at the same time, avoid the problems associated with the farming of transgenic plants. These markers would provide valuable tools both for generating "GM-free" transgenic crops.

According to a first aspect thereof, the object of the present invention is solved by providing an isolated nucleic acid, comprising a continuous sequence of at least 12 bases selected from a) the sequence encoded by SEQ ID NO 1 or 3, b) a sequence having at least 80% sequence identity with SEQ ID NO 1 or 3, c) a sequence encoding the same protein as SEQ ID NO 1 or 3, which is modified due to the degeneracy of the genetic code, or which is modified in order to be adjusted to codon usage of a host, d) an orthologous sequence of SEQ ID NO 1 or 3, and complementary sequences thereof.

In alternative and preferred embodiments, the isolated nucleic acid according to the present invention, comprises at least one continuous sequence of at least 18, 21, 24 or 36 bases, or at least 50, 100, 200 or 500 bases. The nucleotide sequence can also include one or more restriction sites for cloning of said nucleotide sequence, preferably at the 3' and/or 5' end of said sequence.

The inventors conducted a genetic screen in order to identify Arabidopsis thaliana proteins involved in transcriptional gene silencing of a NOS promoter-driven NPTII reporter gene that is induced by double-stranded promoter RNA. One gene isolated from this screen, At5g26820 (SEQ ID NO 1), is annotated to encode a protein with low similarity to ferroportin1 from *Danio rerio.* Ferroportin has important functions in iron homeostasis. At5g26820 has a 54 amino acid chloroplast transit sequence, and has clearly recognizable orthologs in rice (e.g. *Oryza sativa* hypothetical protein AAU44227, SEQ ID NO 4) and in *Triticum aestivum,* indicating a conservation of this protein in monocotyledons as well as dicotyledons, and in particular of the domain structure(s) thereof.

In contrast to other genes identified in the screen, At5g26820 is not important for promoter silencing. In plants mutant for At5g26820, the target NOSpro is still methylated and silenced, and NPTII activity is not detectable. However, mutant plants are nevertheless kanamycin-resistant indicating a kanamycin resistance mechanism that is independent of NPTII expression. Therefore, the putative chloroplast protein At5g26820 could be important for the uptake of kanamycin into these organelles. If this is true, plants lacking this protein should be "blind" to kanamycin.

Sequence 852 of WO 02/16655 discloses a part of SEQ ID NO 1 as a cold stress-regulated gene. In summary, a collection of 5379 genes is disclosed, without any indication of an involvement in kanamycin resistance.

Accession number NM_122564 (of 09-JUN-2006) discloses the 2131 bp mRNA sequence of the *Arabidopsis thaliana* locus_tag AT5G26820 as a ferroportin-related protein with low similarity to ferroportin 1 of *Danio rerio,* without any indication of an involvement in kanamycin resistance.

Preferred is the isolated nucleic acid according to the present invention, wherein sequence b) as above has at least 90% sequence identity, preferably 95% and most preferred 99% with SEQ ID NO 1 or 3. Related aspects of the invention provide an isolated nucleotide sequence that hybridizes under stringent conditions, preferably highly stringent conditions, to the complement of SEQ ID NO 1 or 3.

A polynucleotide portion of SEQ ID NO 1 or 3 can encode the polypeptide or functional peptide portion thereof, which can be operatively linked to a heterologous promoter. As used herein, reference to a "functional peptide portion of the polypeptide" means a contiguous amino acid sequence of the polypeptide that has the kanamycin-resistance activity of the full length polypeptide, or that presents an epitope unique to the polypeptide.

A polynucleotide portion of the SEQ ID NO 1 or 3 nucleotide sequence also can contain a mutation, whereby upon integrating into the plant cell genome and/or chloroplast, the polynucleotide disrupts (knocks-out) an endogenous plant nucleotide sequence, thereby modulating the responsiveness of said plant cell to kanamycin. Thus, a method of the invention provides a means of producing a transgenic plant having a knock-out phenotype of the inventive nucleotide sequence. Knock-out phenotypes can also be produced by mutating the regulatory sequences of the gene for AT5G26820 or its homologs or orthologs, in order to reduce or inactivate the expression of said gene. Exemplary regulatory sequences include promoter and/or enhancer sequences located in the vicinity of the gene for ATSG26820 or its homologs or orthologs, either upstream and/or downstream of the encoding sequence (see also below).

Thus, the invention provides polynucleotide sequences comprising plant kanamycin resistance genes that are homologs or orthologs, variants, or otherwise substantially similar to the polynucleotide disclosed herein, and having an E value < 1 x 10⁻⁸, which can be identified, for example, by a BLASTN search using the *Arabidopsis* polynucleotide SEQ ID NO 1 or 3 as query sequences.

A polynucleotide sequence of the gene as disclosed herein can be particularly useful as a selection marker of the invention on a variety of plants, including but not limited to, corn (*Zea mays),* Brassica sp. (e.g., *B. napus,* B. rapa, *B. juncea*)*,* alfalfa (*Medicago sativa*)*,* rye *(Secale cereale*)*,* sorghum (*Sorghum bicolor, Sorghum vulgare*)*,* sunflower (*Helianthus annuus*)*,* safflower (Carthamus tinctorius), wheat (*Triticum aestivum*)*,* soybean (Glycine max), tobacco (Nicotiana tabacum), potato (*Solanum tuberosum*), peanuts (*Rachis hypogaea*)*,* cotton (Gossypium barbadense, Gossypium hirsutum), sweet potato (*Ipomoea batatus*), cassava (Manihot esculenta), coffee (Cofea spp.), coconut (*Cocos nucifera*)*,* pineapple (Ananas comosus), citrus trees (*Citrus spp.*)*,* cocoa (Theobroma cacao), tea (Camellia sinensis), banana (Musa spp.), avocado (Perseaultilane), fig (Ficuscasica), guava(Psidium guava), mango (Mangifera indica), olive (Olea europaea), papaya (Carica papaya), cashew(Anacardium occidentale), macadamia (Macadamia integrifolia),ahnond (Prunus amygdalus), sugar beets (Beta vulgaris), sugarcane (Saccharum spp.), oats, duckweed (Lemna), barley, tomatoes (Lycopersicon esculentum), lettuce (e. g., Lactuca sativa), green beans (Phaseolus vulgaris), lima beans (Phaseoluslimensis), peas (Lathyrus spp.), and members of the genus Cucumis such as cucumber (C sativus), cantaloupe (C. cantalupensis), and musk melon(C. melo). Ornamentals such as azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbia pulcherrima), and chrysanthemum are also included. Additional ornamentals within the scope of the invention include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia. Conifers that may be employed in practicing the present invention include, for example, pines such as loblolly pine (Pinus taeda), slash pine (Pinus elliotii), ponderosa pine (Pinus ponderosa), lodgepole pine (Pinus contorta), and Monterey pine (Pinus radiata), Douglas-fir (Pseudotsuga menziesii); Western hemlock (Tsugaultilane); Sitka spruce (Picea glauca); redwood (Sequoia sempervirens); true firs such as silver fir (Abies amabilis) and balsam fir (Abies balsamea); and cedars such as Western red cedar (Thuja plicata) and Alaska yellow-cedar (Chamaecyparis nootkatensis).

The percentage of identity between the substantially similar nucleotide sequence and SEQ ID NO 1 or 3 (or parts thereof) as the reference sequence desirably is at least 80%, preferably at least 90%, more preferably at least 95%, still more preferably at least 99% and including 100%. A nucleotide sequence is "substantially similar" to the reference nucleotide sequence if it hybridizes to the reference nucleotide sequence in 7% sodium dodecyl sulfate (SDS), 0.5 M NaPO₄, 1 mM EDTA at 50°C with washing in 2 X SSC, 0.1% SDS at 50°C, more desirably in 7% sodium dodecyl sulphate (SDS), 0.5 M NaPO₄, 1 mM EDTA at 50°C with washing in 1 X SSC, 0.1% SDS at 50°C (stringent conditions), more desirably still in 7% sodium dodecyl sulfate (SDS), 0.5 M NaP04, 1 mM EDTA at 50°C with washing in 0.5 X SSC, 0.1% SDS at 50°C (high stringency).

Preferred is an isolated nucleic acid according to the present invention, wherein said nucleic acid is selected from mRNA, ss-RNA, ds-RNA, ss-DNA, ds-DNA, si-RNA, cNA, and PNA. An antisense polynucleotide, ribozyme or triplexing agent is complementary to a target sequence, which can be a DNA or RNA sequence, for example, messenger RNA, and can be a coding sequence, a nucleotide sequence comprising an intron-exon junction, a regulatory sequence such as a Shine-Delgarno-like sequence, or the like.

The inventors also could phenocopy the original At5g26820 mutant by RNA interference (RNAi): Wild-type plants were transformed with a construct, that encompassed the region coding for the chloroplast transit peptide as a transcribed inverted repeat but that did not contain a kanamycin resistance gene. Consequently, siRNAs from that region are produced in plants transformed with this construct, which further results in the destabilization of endogenous homologous mRNA by RNAi and kanamycin resistance. Indeed, plants transgenic for this construct could be selected on kanamycin agar. Therefore, targeted knock-down of At5g26820 results in kanamycin resistance in the absence of a kanamycin resistance gene.

Included in the scope of the isolated nucleic acid according to the present invention are also nucleic acids according to the present invention that contain introns. Usually this will refer to chromosomically encoded genes. At5g26820 is nuclear-encoded, the gene product will translocate to the chloroplast.

The terms "polynucleotide", "oligonucleotide," and "nucleic acid sequence" are used interchangeably herein to refer to a polymeric (two or more monomers) form of nucleotides of any length, either ribonucleotides or deoxyribonucleotides. Although nucleotides are usually joined by phosphodiester linkages, the term also includes polymers containing neutral amide backbone linkages composed of aminoethyl glycine units. The terms are used only to refer to the primary structure of the molecule. Thus, the term includes double stranded and single stranded DNA molecules as above. It will be recognized that such polynucleotides can be modified, for example, by including a label such as a radioactive, fluorescent or other tag, by methylation, by the inclusion of a cap structure, by containing a substitution of one or more of the naturally occurring nucleotides with a nucleotide analogue, by containing an internucleotide modification such as having uncharged linkages (e.g., methyl phosphonates, phosphotriesters, phosphoramidates, carbamates, or the like), by containing a pendant moiety such as a protein (e.g., a nuclease, toxin, antibody, signal peptide, poly-L-lysine, or the like), by containing an intercalator such as acridine or psoralen, by containing a chelator, which can be a metal such as boron, an oxidative metal, or a radioactive metal, by containing an alkylator, or by having a modified linkage (e.g., an alpha anomeric nucleic acid).

Quite recently, another group published that over-expression of an ABC transporter confers kanamycin resistance to transgenic plants by pumping the drug into vacuoles (Mentewab A, Stewart CN Jr.: Overexpression of an Arabidopsis thaliana ABC transporter confers kanamycin resistance to transgenic plants. Nat Biotechnol. 2005 Sep; 23(9):1177-80. Epub 2005 Aug 21. Erratum in: Nat Biotechnol. 2005 Oct; 23(10):1315). The authors also discuss the potential of ABC transporter over-expression as a novel marker in biotechnology. The mechanism of resistance conferred by protein over-expression, however, is likely different from the present invention: it is the loss-of function of At5g26820 which causes kanamycin resistance and not its over-expression. Also, although At5g26820 might have transport function, it is not an ABC transporter.

Only one mutant allele of At5g26820 was originally identified in the above screen. This allele results in an amino acid change at position 323 of the protein from valine to methionine. Transformation of mutant plants by a genomic fragment harboring the wild-type At5g26820 gene results in functional complementation, giving rise to kanamycin-sensitive progeny plants again.

Yet another preferred aspect of the present invention thus relates to an inactivation cassette, comprising a nucleic acid according to the present invention as above. More preferably, the inactivation cassette according to the present invention comprises a nucleic acid comprising a functionally inactivating mutation, deletion, insertion, repeat, inversion, an inverted repeat of a polynucleotide homologous to At5g26820 and/or an inverted repeat of upstream regulatory sequences of At5g26820 or homologous genes as described above, preferably of up to about 1500bp upstream of the translational start site. In a preferred embodiment of the present invention, said inactivation cassette is a t-DNA. Transferred DNA (t-DNA) is transferred as a single-stranded derivative from *Agrobacterium* to the plant cell nucleus and is described, for example, in Tinland et al. (Tinland B, Hohn B, Puchta H. *Agrobacterium tumefaciens* transfers single-stranded transferred DNA (t-DNA) into the plant cell nucleus, Proc Natl Acad Sci USA, 1994 Aug 16; 91(17):8000-4.)

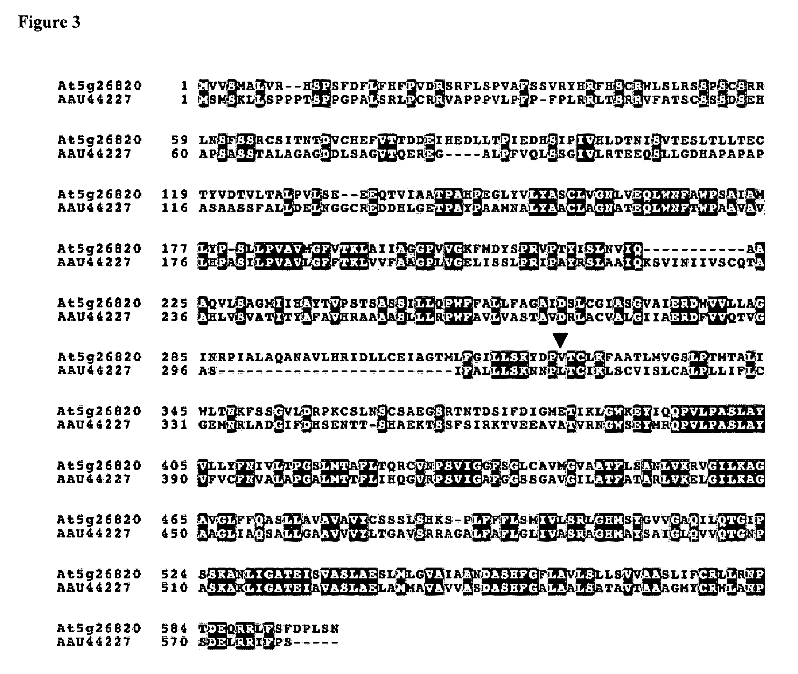

Preferred is the inactivation cassette according to the present invention, wherein said inactivating mutation encodes for a valine323 to methionine323 amino acid exchange in SEQ ID No. 2, or for an analogous mutation in SEQ ID No. 4 (see Figure 3), or their respective homologs or orthologs.

A further aspect of the present invention is then directed at a vector comprising a polypeptide/protein according to the present invention and/or a nucleic acid according to the present invention. A vector within the meaning of the present invention is a protein or a nucleic acid or a mixture thereof which is capable of being introduced or of introducing the proteins and/or nucleic acid comprised into a cell. It is preferred that the proteins encoded by the introduced nucleic acid are expressed within the cell upon introduction of the vector.

In a preferred embodiment the vector of the present invention comprises recombinant vectors, plasmids, phagemids, phages, cosmids, viruses, in particular but not limited to virus-derived amplicon vectors, potato virus X based vectors, tobacco rattle virus based vectors, geminivirus-based vectors such as cabbage leaf curl virus and barley stripe mosaic virus based vectors and vectors based on satellite viruses (reviewed in Curtin, S.J., Wang, M.-B., Watson, J.M., Roffey, P., Blanchard, C.L. and Waterhouse, P.M.. (2007), chapter 12, p 291-332 in "Rice Functional Genomics; Challenges, Progress and Prospects". Upadhyaya, Narayana M. (Ed.), ISBN: 978-0-387-48903-2), virosomes, "naked" DNA liposomes, and nucleic acid coated particles, in particular gold spheres.. Liposomes are usually small unilamellar or multilamellar vesicles made of cationic and anionic lipids, for example, by ultrasound treatment of liposomal suspensions. The DNA can, for example, be ionically bound to the surface of the liposomes or internally enclosed in the liposome. Suitable lipid mixtures are known in the art and comprise, for example, DOTMA (1,2-dioleyloxpropyl-3-trimethylammoniumbromide) and DPOE (dioleoylphosphatidylethanolamine) which both have been used on a variety of cell lines.

The term "recombinant nucleic acid molecule" refers to a polynucleotide produced by human intervention. A recombinant nucleic acid molecule can contain two or more nucleotide sequences that are linked in a manner such that the product is not found in a cell in nature. In particular, the two or more nucleotide sequences can be operatively linked and, for example, can encode a fusion polypeptide, or can comprise a nucleotide sequence and a regulatory element. A recombinant nucleic acid molecule also can be based on, but different, from a naturally occurring polynucleotide, for example, a polynucleotide having one or more nucleotide changes such that a first codon, which normally is found in the polynucleotide, is replaced with a degenerate codon that encodes the same or a conservative amino acid, or such that a sequence of interest is introduced into the polynucleotide, for example, a restriction endonuclease recognition site or a splice site, a promoter, a DNA replication initiation site, or the like.

Preferred is a recombinant vector according to the present invention, which is an expression vector, optionally comprising one or more genes to be expressed. Preferably, said expression is driven by a regulatory element (or elements). As used herein, the term "regulatory element" means a nucleotide sequence that, when operatively linked to a coding region of the gene as above, effects transcription of the coding region such that a ribonucleic acid (RNA) molecule is transcribed from the coding region. A regulatory element generally can increase or decrease the amount of transcription of a nucleotide sequence, for example, a coding sequence, operatively linked to the element with respect to the level at which the nucleotide sequence would be transcribed absent the regulatory element. Regulatory elements are well known in the art and include promoters, enhancers, silencers, inactivated silencer intron sequences, 3'-untranslated or 5'-untranslated sequences of transcribed sequence, for example, a poly-A signal sequence, or other protein or RNA stabilizing elements, or other gene expression control elements known to regulate gene expression or the amount of expression of a gene product. A regulatory element can be isolated from a naturally occurring genomic DNA sequence or can be synthetic, for example, a synthetic promoter.

Regulatory elements can be constitutively expressed regulatory element, which maintain gene expression at a relative level of activity (basal level), or can be regulated regulatory elements. Constitutively expressed regulatory elements can be expressed in any cell type, or can be tissue specific, which are expressed only in particular cell types, phase specific, which are expressed only during particular developmental or growth stages of a plant cell, or the like. A regulatory element such as a tissue specific or phase specific regulatory element or an inducible regulatory element useful in constructing a recombinant polynucleotide or in a practicing a method of the invention can be a regulatory element that generally, in nature, is found in a plant genome. However, the regulatory element also can be from an organism other than a plant, including, for example, from a plant virus, an animal virus, or a cell from an animal or other multicellular organism.

A regulatory element useful for practicing method of the present is a promoter element. Useful promoters include, but are not limited to, constitutive, inducible, temporally regulated, developmentally regulated, spatially-regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression. An inducible promoter is a promoter that provides for the turning on and off of gene expression in response to an exogenously added agent, or to an environmental or developmental stimulus. A bacterial promoter such as the Pfade promoter can be induced to varying levels of gene expression depending on the level of isothiopropyl galactoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

The choice of promoter will vary depending on the temporal and spatial requirements for expression, and also depending on the target species. In some cases, expression in multiple tissues is desirable. While in others, tissue-specific, e.g., leaf-specific, seed-specific, petal-specific, anther-specific, or pith-specific, expression is desirable. Although many promoters from dicotyledons have been shown to be operational in monocotyledons and vice versa, ideally dicotyledonous promoters are selected for expression in dicotyledons, and monocotyledonous promoters for expression in monocotyledons. There is, however, no restriction to the origin or source of a selected promoter. It is sufficient that the promoters are operational in driving the expression of a desired nucleotide sequence in the particular cell.

Other sequences that have been found to enhance gene expression in transgenic plants include intron sequences (e. g., from Adh I, bronze 1, actin 1, actin 2 (WO 00/760067), or the sucrose synthase intron) and viral leader sequences (e.g., from TMV, MCMV and AMV). For example, a number of non-translated leader sequences derived from viruses are known to enhance expression. Specifically, leader sequences from tobacco mosaic virus (TMV), maize chlorotic mottle virus (MCMV), and alfalfa mosaic virus (AMV) have been shown to be effective in enhancing expression (e.g., Gallie et al., 1987;Skuzeski et al., 1990). Other leaders known in the art include but are not limited topicornavirus leaders, for example, EMCV leader (encephalomyocarditis virus 5'-non-coding region; Elroy-Stein et al., 1989); potyvirus leaders, for example, TEV leader (tobacco etch virus); MDMV leader (maize dwarf mosaic virus); human immunoglobulin heavy chain binding protein (BiP) leader, (Macejak et al., 1991); untranslated leader from the coat protein mRNA of AMV (AMV RNA 4; Jobling et al., 1987), TMV (Gallie et al.,1989), and MCMV (Lommel et al., 1991; see also, della Cioppa et al., 1987).

The present invention is directed at an isolated polypeptide comprising the same or substantially the same amino acid sequence selected from SEQ ID NO: 2 or 4, or a splice variant or a salt thereof. A protein having substantially the same amino acid sequence comprises proteins with at least about 80%, preferably at least about 90%, more preferably at least about 95%, preferably at least about 96%, more preferably at least about 97%, more preferably with at least about 98% and most preferably with at least about 99% amino acid sequence identity. The amino acid exchanges are preferably so called conservative changes meaning substitutions of, for example, a polar amino acid residue by another polar amino acid residue, of a acidic amino acid residue by another acidic amino acid residue or of a basic amino acid residue by another basic amino acid residue.

Although the overall identity of the polypeptides according to SEQ ID NO: 2 or 4 is rather low (about 44%), a domain-structure was found (inverted letters) which serves as a basis for an identification of additional inventive polypeptide sequences in other plants. A protein having substantially the same amino acid sequence therefore also comprises proteins with at least about 80%, preferably at least about 90%, more preferably at least about 95%, preferably at least about 96%, more preferably at least about 97%, more preferably with at least about 98% and most preferably with at least about 99% amino acid sequence identity inside the domain regions as indicated in Figure 3 (inverted letters).

Proteins having substantially the same amino acid within the meaning of this invention exhibit in a preferred embodiment kanamycin resistance activity, if the protein is mutated, inactivated or knocked-down, kanamycin resistance will result. The proteins employed in a respective assay can either be purified from cells or can be recombinantly expressed and purified by methods well known in the art.

In one embodiment of the present invention the protein comprises at least one fragment of the amino acid sequence selected from SEQ ID NO: 2 or 4. A fragment within the meaning of the present invention refers to one of the proteins according to SEQ ID NO: 1 or 3 bearing at least one N-terminal, C-terminal and/or internal deletion. The resulting fragment has a length of at least about 50, preferably of at least about 100, more preferably of at least about 150, more preferably of at least about 200, more preferably of at least about 250, more preferably of at least about 300, more preferably of at least about 350 and most preferably of at least about 400 amino acids.

The fragment itself has preferably an amino acid sequence identity with the amino acid sequence selected from SEQ ID NO: 2 or 4 of at least about 80%, preferably of at least about 90%, more preferably of at least about 95%, more preferably of at least about 98%, more preferably of at least about 99% and most preferably of 100%.

In a further aspect the present invention is directed at an antibody directed against a protein of the present invention. An antibody that is specific for the polypeptide, wherein said antibody preferably functionally inhibits said polypeptide. The term "antibody" comprises monoclonal and polyclonal antibodies and binding fragments thereof, in particular Fc-fragments as well as so called "single-chain-antibodies" (Bird R. E. et al (1988) Science 242:423-6) and diabodies (Holliger P. et al (1993) Proc. Natl. Acad. Sci. U.S.A. 90:6444-8). The term "antibody" comprises also recombinantly produced and optionally modified antibodies or antigen-binding parts thereof, such as e.g. chimeric antibodies, humanized antibodies, multifunctional antibodies, bi- or oligo specific antibodies, single stranded antibodies, F(ab)- or F(ab)₂-fragments (see e.g. EP-B1-0 368 684, US 4,816,567, US 4,816,397, WO 88/01649, WO 93/06213, WO 98/24884).

A further aspect the present invention is directed at a method for producing an antibody, preferably a monoclonal and polyclonal antibody, wherein a antibody producing organism is immunized with a polypeptide of the present invention or functional fragment thereof with at least 6 amino acids, preferably with at least 8 amino acids, in particular with at least 12 amino acids or a nucleic acid of the present invention. Monoclonal antibodies can be produced, for example, according to the known method of Winter and Milstein (1991, Nature 349: 293-299).

Yet another aspect the present invention is then directed at an expression cassette or vector which comprises a nucleic acid encoding the antibody according to the present invention. Respective vectors are as described above.

Yet another aspect the present invention then relates to a method for producing a recombinant kanamycin resistant plant, comprising functionally inactivating the polypeptide according to the present invention. The invention provides a genetically modified plant, which can be a transgenic plant, that is resistant to kanamycin. As used herein, the term "resistant", when used in reference to a kanamycin stress, means that the particular plant, when exposed to a stress condition, shows less of an effect, or no effect, in response to said condition as compared to a corresponding reference plant (naturally occurring wild-type plant or a plant not containing a construct of the present invention). Preferred is a method according to the present invention, wherein said functional inactivation comprises inhibiting the expression and/or activity of said protein. Interestingly, the effect of At5g26820 knock-down is very specific for kanamycin. Knock-down plants are not resistant to other aminoglycoside antibiotics, such as hygromycin and gentamycin. Thus, preferred is a method according to the present invention, wherein said kanamycin is selected from A, B, C, combimicins, and derivatives thereof.

Still another aspect the present invention then relates to a method for producing a recombinant kanamycin resistant plant, comprising introducing an inactivation cassette according to the invention as above or a vector according to the invention as above into said plant. Methods for creating such recombinant plants are well known to the person of skill. Still another aspect the present invention then relates to a recombinant or transgenic plant, that is produced according to such a method. The methods of the invention provide genetically modified plant cells, which can contain, for example, a coding region, or peptide portion thereof, of the kanamycin resistance gene operatively linked to a heterologous inducible regulatory element; or the kanamycin resistance gene regulatory element operatively linked to its nucleotide sequence encoding a polypeptide of interest. In such a plant, the expression from the inducible regulatory element can be effected by exposing the plant cells to an inducing agent in any of numerous ways depending, for example, on the inducible regulatory element and the inducing agent. Similarly, a transgenic plant containing a metallothionein promoter can be exposed to metal ions such as cadmium or copper by watering the plants with a solution containing the inducing metal ions, or can be planted in soil that is contaminated with a level of such metal ions that is toxic to most plants. Still another aspect the present invention then relates to a transgenic plant produced according to the present invention. Preferably, said plant expresses at least one transgene, wherein said gene can be selected from the nucleic acid(s) encoding for At5g26820 (as described above), and additional genes that are introduced and selected for.

Preferably, said recombinant or transgenic plant according to the invention is a monocotyledonous or dicotyledonous plant, for example as disclosed above. Since At5g26820 is also present in monocotyledonous plants, which include the majority of ecologically relevant crop plants, the same strategy could also be applied for the engineering of said crops.

According to yet another aspect thereof, the object of the present invention is solved by providing a method of identifying a compound that modulate the expression and/or biological activity of At5g26820 in a cell, comprising the steps of a) contacting a cell expressing At5g26820 with at least one potentially interacting compound, and b) measuring the modulation of the expression and/or biological activity of At5g26820 in said cell. Preferred is a method according to the present invention, wherein said compound as identified is an inhibitor of the expression and/or biological activity of At5g26820 in said cell.

This method is suitable for the determination of compounds that can interact with At5g26820 and to identify, for example, inhibitors, activators, competitors or modulators of At5g26820, in particular inhibitors, activators, competitors or modulators of the enzymatic activity of At5g26820. Another aspect is directed at compounds that modulate the expression of At5g26820 in a cell/in cells.

The term "contacting" in the present invention means any interaction between the potentially binding substance(s) with At5g26820, whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip and At5g26820 (or a functional part thereof) is subsequently contacted with such a chip.

The At5g26820 employed in a method of the present invention can be a full length protein or a fragment with N/C-terminal and/or internal deletions. Preferably the fragment is either an N-terminal fragment comprising the enzymatic region of the polypeptide or a C-terminal fragment comprising the cytoplasmatic region, depending on whether potentially interacting compounds are sought that specifically interact with the N- or C-terminal fragment.

The potentially binding substance, whose binding to At5g26820 is to be measured, can be any chemical substance or any mixture thereof. For example, it can be a substance of a peptide library, a combinatory library, a cell extract, in particular a plant cell extract, a "small molecular drug", a protein and/or a protein fragment.

Measuring of binding of the compound to At5g26820 can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of either At5g26820 or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to At5g26820 or At5g26820 fragments. The effect of the binding of the compound or the activity of At5g26820 can also be measured indirectly, for example, by assaying the level of the kanamycin resistance activity of At5g26820 after binding, and the like.

The inventors' work sets the ground for using At5g26820 knock-down as a novel marker for selecting transgenic plants. This strategy would work without the use of antibiotic resistance genes as markers but would still allow conventional selection of the transgenic plants on antibiotic-containing media.

The use of antibiotic resistance genes is very controversial in public and is used as a heavy argument by activists (and opinion-makers) against "green biotechnology", since they fear the horizontal gene transfer from transgenic plants to bacteria and, ultimately, the generation of antibiotic resistant pathogenic bacteria. Second, the strategy involves the knock-down of an endogenous gene rather than the (over-) expression of a foreign protein. Marker-gene over-expression in transgenic plants and the potential development of allergies by their consumers is another, very often used argument of activists against "green biotechnology".

In summary, the inventors have identified a novel mechanism how plants can gain resistance to a frequently used antibiotic drug. This novel mechanism could be used in the commercial production of biologically engineered plants without the disadvantages of using over-expressed antibiotic resistance genes as transgenic markers.

The following figures, sequences, and examples merely serve to illustrate the invention and should not be construed to restrict the scope of the invention to the particular embodiments of the invention described in the examples. All references cited in the text are hereby incorporated in their entirety by reference.

SEQ ID NO 1 shows the inventive nucleotide sequence of the *Arabidopsis thaliana* gene for At5g26820.

SEQ ID NO 2 shows the inventive polypeptide sequence *of Arabidopsis thaliana* At5g26820.

SEQ ID NO 3 shows the inventive nucleotide sequence of the *Oryza sativa* gene for At5g26820 (AAU44227).

SEQ ID NO 4 shows the inventive polypeptide sequence of *Oryza sativa* protein AAU44227.

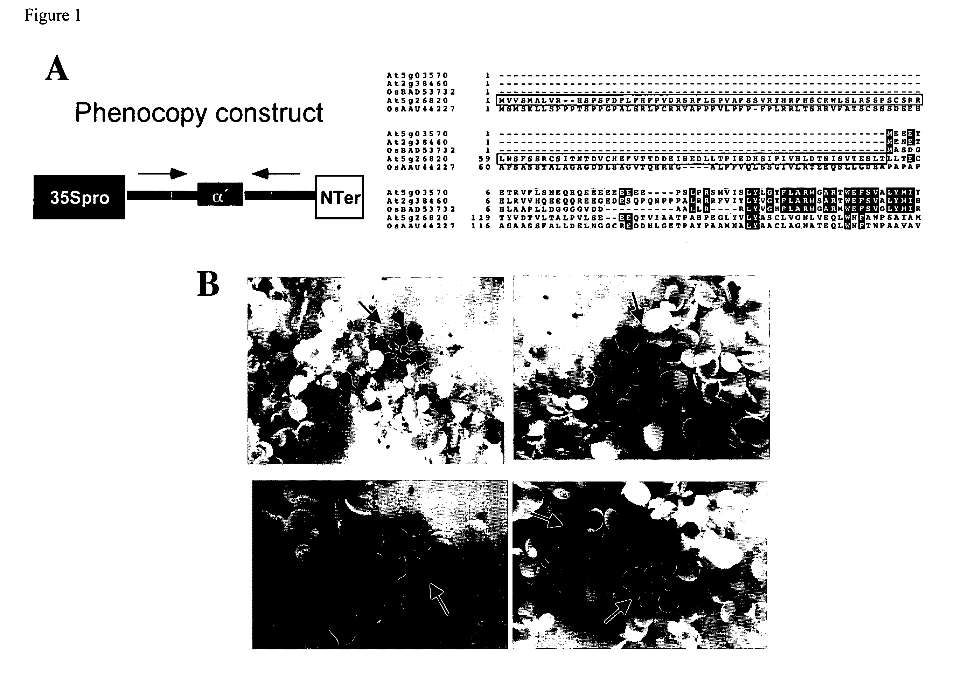

**Figure 1A****:** The left panel shows the phenocopy construct that was used to knock-down At5g26820. The DNA encoding the putative chloroplast transit peptide is shown in red and was cloned in an inverted repeat configuration indicated by the arrows with the repeat units separated by an unrelated spacer (□'). The black bars denote an intron located within the region encoding the transit peptide. The inverted repeat is expressed from the constitutive 35Spromoter and terminated by the NOS terminator. The right panel shows the identity of the transit peptide boxed in red. **1B:** Plants transgenic for the phenocopy construct were successfully selected on nutrient agar containing 35mg/l kanamycin A (plants indicated by arrows). For comparison, *Arabidopsis* plants transgenic for a kanamycin-resistance gene, such as NPTII, are usually selected with 50mg/l kanamycin A.

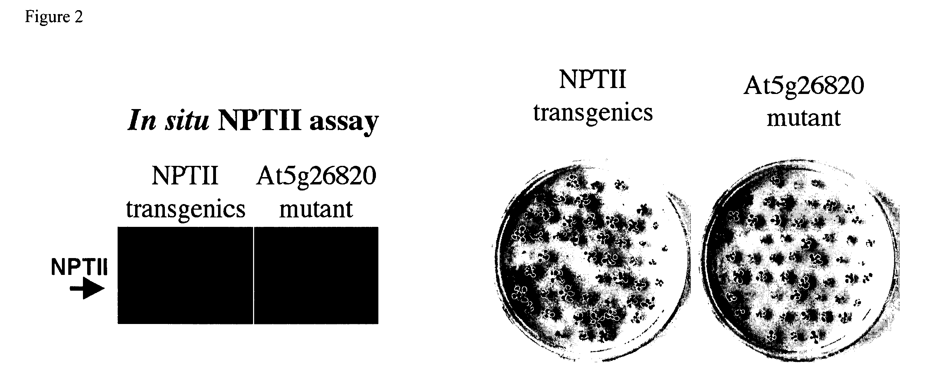

**Figure 2****:** The left panel shows measurement of NPTII activity in plants transgenic for an NPTII kanamycin-resistance gene and in plants having a mutation in At5g26820. Although mutant plants do not show NPTII activity (left panel), they exhibit solid kanamycin resistance when grown on nutrient agar with 35mg/l kanamycin A (right panel).

**Figure 3**: shows an alignment of the inventive polypeptide sequence of *Arabidopsis thaliana* with *Oryza sativa.* Identical amino acid residues are boxed in black, similar amino acids are shaded in grey. The red arrowhead denotes valine 323 which is changed to methionine in the mutant allele and which, in the rice protein, is a leucine. Although the overall identity of the polypeptides is rather low, a domain-structure can be found (inverted letters) which can form the basis for an identification of additional inventive polypeptide sequences in other plants.

### Examples

The inventors conducted a genetic screen in order to identify Arabidopsis proteins involved in transcriptional gene silencing that is induced by double-stranded promoter RNA.

A two-component transgene system was used as a basis for this screen:
a) The first transgene (i.e. target) contains the kanamycin resistance gene (NPTII) as a reporter gene, which is under control of the moderately strong, constitutive nopaline synthase promoter (NOSpro) from Agrobacterium.
b) The second transgene (i.e. silencer) provides constitutive transcription of a NOSpro inverted repeat, thus resulting in the production of NOSpro dsRNA that is further processed to NOSpro small RNAs of 21-25 nucleotides in length by a Dicer-like activity.

Plants harboring both, the target and silencer transgenes, are kanamycin sensitive due to cytosine methylation and transcriptional inactivation of the target NOSpro that controls NPTII expression. The inactivation of the reporter gene is triggered by sequence homologous double-stranded (ds) RNA originating from the silencer.

Plants homozygous for silencer and target were mutagenized and the M2 progeny of these plants was selected on agar containing kanamycin. Plants, which were again able to grow on kanamycin despite the presence of the silencer, were considered as good candidates for being defective in important factors controlling RNA-mediated transcriptional gene silencing.

One gene isolated from this screen, At5g26820, is annotated to encode a protein with low similarity to ferroportin 1 from *Danio rerio.* Ferroportin has important functions in iron homeostasis. Three genes in the *Arabidopsis* genome have low homology to ferroportin: At2g38460, At5g03750 and At5g26820. At5g26820 has a 54aa chloroplast transit sequence identified by the Chloro P1.1 software (http://www.cbs.dtu.dk), which does not occur within the other two Arabidopsis proteins. Moreover, At2g38460 and At5g03750 proteins are more closely related to each other than to the At5g26820 protein. At5g26820 has clearly recognizable orthologs in rice (Os AAU44227) and in *Triticum aestivum,* indicating a conservation of this protein in monocotyledons as well as dicotyledons.

It was surprisingly found that, in contrast to other genes identified in the screen, At5g26820 is not important for promoter silencing. In plants mutant for At5g26820, the target NOSpro is still methylated and silenced, and NPTII activity is not detectable. However, mutant plants are nevertheless kanamycin-resistant indicating a kanamycin resistance mechanism that is independent of NPTII expression. Therefore, the putative chloroplast protein At5g26820 could be important for the uptake of kanamycin into these organelles. Thus, plants lacking (or haying a reduced amount of) this protein should be "blind" to kanamycin.

Only one mutant allele was identified in the screen. This allele results in an amino acid change at position 323 of the protein from valine to methionine. Transformation of mutant plants by a genomic fragment harboring the wild-type At5g26820 gene results in functional complementation, giving rise to kanamycin-sensitive progeny plants again.

The inventors could phenocopy the original mutant by RNA interference (RNAi). Wild-type plants were transformed with a construct, that encompassed the region coding for the chloroplast transit peptide as a transcribed inverted repeat but that did not contain a kanamycin resistance gene. Consequently, siRNAs from that region are produced in plants transformed with this construct, which further resulted in the destabilization of endogenous homologous mRNA by RNAi and kanamycin resistance.

Indeed, plants transgenic for this construct could be selected on kanamycin agar. Therefore, targeted knock-down of At5g26820 results in kanamycin resistance in the absence of a kanamycin resistance gene. Interestingly, the effect of At5g26820 knock-down is very specific for kanamycin. Knock-down plants are not resistant to other aminoglycoside antibiotics, such as hygromycin and gentamycin.

## Claims

1. An isolated nucleic acid, comprising a continuous sequence of at least 12 bases selected from
a) SEQ ID NO 1 or 3,
b) a sequence having at least 80% sequence identity with SEQ ID NO 1 or 3,
c) a sequence encoding the same protein as SEQ ID NO 1 or 3, which is modified due to the degeneracy of the genetic code, or which is modified in order to be adjusted to codon usage of a host,
d) an orthologous sequence of SEQ ID NO 1 or 3, and
complementary sequences thereof.

2. The isolated nucleic acid according to claim 1, wherein sequence b) has at least 90% sequence identity, preferably 95% and most preferred 99% with SEQ ID NO 1 or 3.

3. The isolated nucleic acid according to claim 1 or 2, wherein said nucleic acid contains introns.

4. The isolated nucleic acid according to any of claims 1 to 3, wherein said nucleic acid is selected from mRNA, ss-RNA, ds-RNA, ss-DNA, ds-DNA, si-RNA, RNAi, cNA, and PNA.

5. An inactivation cassette, comprising a nucleic acid according to any of claims 1 to 4.

6. The inactivation cassette according to claim 5, wherein said nucleic acid comprises a functionally inactivating mutation, deletion, insertion, repeat, and/or inversion.

7. The inactivation cassette according to claim 5 or 6, which is a t-DNA.

8. The inactivation cassette according to claim 5 or 6, wherein said mutation encodes for a valine323 to methionine323 mutation of SEQ ID NO 2.

9. A recombinant vector, comprising a nucleic acid according to any of claims 1 to 8.

10. The recombinant vector according to claim 9, which is an expression vector, optionally comprising one or more genes to be expressed.

11. A polypeptide, encoded by a nucleic acid according to any of claims 1 to 4, in particular a polypeptide according to SEQ ID NO 2 or 4.

12. An antibody that is specific for the polypeptide according to claim 11, wherein said antibody functionally inhibits said polypeptide.

13. An expression cassette or vector, comprising a nucleic acid encoding the antibody according to claim 12.

14. A method for producing a recombinant kanamycin resistant plant, comprising functionally inactivating the polypeptide according to claim 11.

15. The method according to claim 14, wherein said functional inactivation comprises inhibiting the expression and/or activity of said protein.

16. The method according to claim 14 or 15, wherein said kanamycin is selected from A, B, C, combimicins, and derivatives thereof.

17. The method according to any of claims 12 to 16, comprising introducing an inactivation cassette according to any of claims 5 to 8 or a vector according to claim 9, 10 or 13 into said plant.

18. A recombinant or transgenic plant, produced according to any of claim 14 to 17.

19. The transgenic plant according to claim 18, which further expresses at least one transgene.

20. The recombinant or transgenic plant according to claim 18 or 19, which is a monocotyledonous or dicotyledonous plant.
